(19) Europäisches Patentamt
European Patent Office
Office européen des brevets

(11) Numéro de publication: **0 178 961**
**B1**

(12) **FASCICULE DE BREVET EUROPEEN**

(45) Date de publication du fascicule du brevet: **08.08.90**

(21) Numéro de dépôt: **85401730.8**

(22) Date de dépôt: **06.09.85**

(51) Int. Cl.$^5$: **A 61 K 31/55** // (A61K31/55, 31:505)

(54) **Compositions pharmaceutiques à base de diltiazem et d'alfuzosine.**

(30) Priorité: **14.09.84 FR 8414088**

(43) Date de publication de la demande:
**23.04.86 Bulletin 86/17**

(45) Mention de la délivrance du brevet:
**08.08.90 Bulletin 90/32**

(84) Etats contractants désignés:
**BE CH DE FR GB IT LI LU NL SE**

(56) Documents cités:
**FR-A-2 421 888**

**Le dossier contient des informations techniques présentées postérieurement au dépôt de la demande et ne figurant pas dans le présent fascicule.**

(73) Titulaire: **SYNTHELABO**
**58, rue de la Glacière**
**F-75621 Paris Cedex 13 (FR)**

(72) Inventeur: **Cavero, Icilio**
**8, rue Gabriel Fauré**
**F-94000 Creteil (FR)**
Inventeur: **Hicks, Peter E.**
**15, rue des Amandiers**
**F-94230 Cachan (FR)**
Inventeur: **Langer, Salomon Z.**
**92, rue Jouffroy**
**F-75017 Paris (FR)**

(74) Mandataire: **Thouret-Lemaitre, Elisabeth et al**
**Service Brevets - SYNTHELABO 58, rue de la Glacière**
**F-75621 Paris Cédex 13 (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

**Description**

Le présente invention a pour objet des compositions pharmaceutiques à base de diltiazem et d'alfuzosine, destinées au traitement de maladies cardiovasculaires, plus particulièrement de l'hypertension quelle que soit son origine.

Le diltiazem, de formule

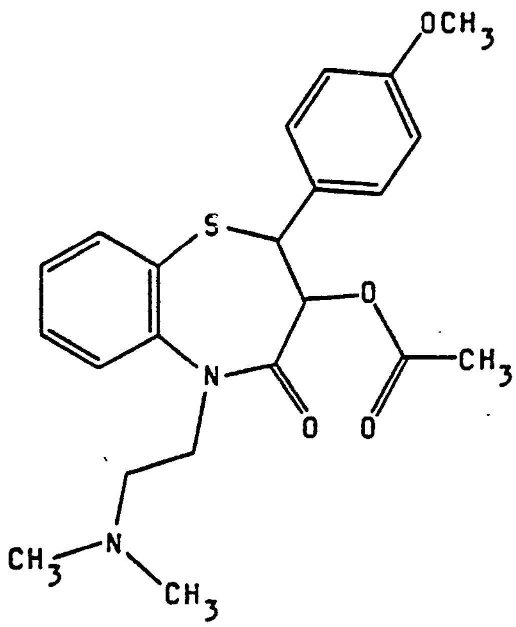

est connu pour ses propriétés antianginales et antihypertensives.

L'alfuzosine, de formule

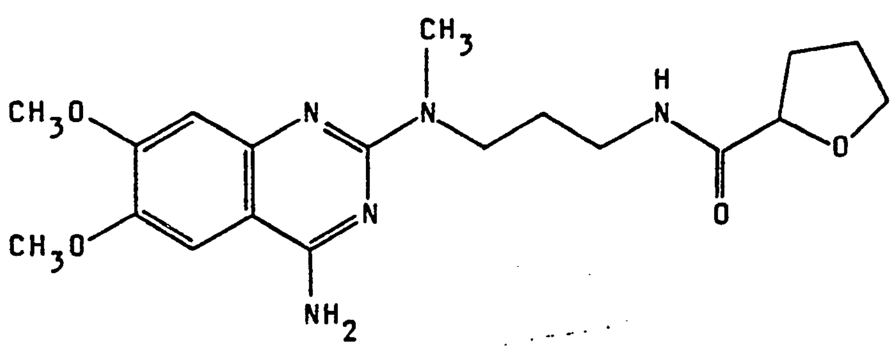

possède également des propriétés antihypertensives.

La demanderesse a constaté que, de manière surprenante, il éxiste un effet de synergie important entre les propriétés antihypertensives des deux composés lorsqu'on les associe.

Les compositions pharmaceutiques de l'invention ont été soumises à une série d'essais pharmacologiques qui révèlent leurs intéressantes propriétés dans le domaine cardiovasculaire.

Les tests utilisés sont les suivants:

1. Test intraveineux:

Des rats spontanément hypertendus (SHR) âgés der plus de cinq mois sont anesthésiés au pentobarbital sodique (60 mg/kg iv.) et préparés de manière à permettre l'enregistrement de la pression sanguine à partir d'une artère, carotide, cannulée.

On injecte les produits à tester par voie intraveineuse via la veine fémorale.

On infuse alors 10 µg/kg/mn de diltiazem par voie intraveineuse, ou de son solvant (eau distillée contenant 0,09% de chlorure de sodium), pendant 45 mn.

Puis, 10 mn après, on injecte l'alfuzosine à raison de 2 µg/kg/mn, pendant 5 mn.

Les changements maximaux de pression sanguine après injection de diltiazem, d'alfuzosine ou de leur association sont mesurés.

Le diltiazem seul produit une diminution continue de la pression sanguine de 12,6 ± 2,1 mmHg (n = 15).

L'alfuzosine seule produit une diminution de la pression sanguine de 19,7 ± 4,7 mmHg (n = 6) à la fin de l'administration, et de 4,2 ± 1,5 mmHg, 15 mn plus tard.

L'association diltiazem/alfuzosine diminue la pression sanguine de 58,4 ± 7,8 mmHg (n =7) à la fin de l'administration de l'alfuzosine, et 15 mn plus tard la diminution est ensore de 48,7 ± 8,7 mmHg.

2. Test oral:

Des rats mâles vigiles spontanément hypertendus (SHR) âgés de plus de cinq mois sont utilisés dans ce test.

On mesure leur pression artérielle et leur fréquence cardiaque, en continu, à partir de l'artère de la queue cathétérisée.

Lorsque la pression artérielle est stabilisée (après 2 h. environ) les animaux reçoivent, par voie orale, dans un volume total de 5 ml/kg soit 1 mg/kg d'alfuzosine, soit 12,5 mg/kg de diltiazem, soit le placebo (eau distillée + 0,2% de Tween 80), soit l'association diltiazem + alfuzosine.

L'administration orale de 12,5 mg/kg de diltiazem n'a pas d'influence significative sur la pression artérielle ou sur la fréquence cardiaque du rat.

L'alfuzosine (1 mg/kg p.o.) diminue l'égèrement la pression artérielle (−22 ± 4 mmHg après 30 mn; la pression initiale étant de 186 ± 4 mmHg; n = 9). Une heure après le traitement, le paramètre est revenue à la valeur initiale.

L'association alfuzosine/diltiazem provoque une diminution de la pression artérielle.

L'activité hypotensive de l'association est maximale 15 mn après le traitement (− 96 ± 4 mmHg; pression initiale = 195 ± 5 mmHg, n = 11).

Une heure après le traitement, la diminution est encore égale à 70% du maximum. Quatre heures après le traitement la diminution est encore égale à 30% du maximum.

La fréquence cardiaque n'est pas modifiée significativement par chacun des composés ou par l'association.

Les résultats montrent qu'il existe une synergie entre les effets antihypertenseurs de l'alfuzosine et du diltiazem.

Les compositions pharmaceutiques de l'invention peuvent contenir de 25 à 160 mg de diltiazem et de 0,1 à 10 mg d'alfuzosine par unité de prise.

Elles peuvent être présentées sous toute forme appropriée pour l'administration par voie orale ou parentérale, en association avec tout excipient pharmaceutique.

Le posologie quotidienne est telle que l'on administre de 25 à 320 mg de diltiazem et de 0,2 à 20 mg d'alfuzosine.

## Revendications

1. Composition pharmaceutique contenant une association de diltiazem et d'alfuzosine.
2. Composition selon la revendication 1, caractérisée en ce qu'elle contient de 25 à 160 mg de diltiazem et de 0,1 à 10 mg d'alfuzosine par unité de prise.

## Patentansprüche

1. Pharmzeutische Zusammensetzung, die eine kombination von Diltiazem und Alfuzosin enthält.
2. Zusammensetzung nach Anspruch 1, dadurch gekennzeichnet, daß sie 25 bis 160 mg Diltiazem und 0,1 bis 10 mg Alfuzosin pro Dosierungseinheit enthält.

## Claims

1. Pharmaceutical composition containing a combination of diltiazem and alfuzosin.
2. Composition according to Claim 1, characterized in that it contains from 25 to 160 mg of diltiazem and from 0.1 to 10 mg of alfuzosin per dose unit.